# EUROPÄISCHE PATENTANMELDUNG

(11) **EP 4 505 894 A1**
(43) Veröffentlichungstag der Anmeldung: **12.02.2025**
(21) Anmeldenummer: 24192485.1
(22) Anmeldetag: 02.08.2024
(51) Int. Cl.: A42B 3/04

(54) **SCHUTZHELM**

(30) Priorität: 08.08.2023 DE 102023121097
(71) Anmelder: ABUS August Bremicker Söhne KG, 58300 Wetter-Volmarstein (DE)
(72) Erfinder:
(74) Vertreter: Manitz Finsterwald Patent- und Rechtsanwaltspartnerschaft mbB

(57) **Zusammenfassung**

Bei einem Schutzhelm, insbesondere einem Sporthelm, mit einer stoßabsorbierenden Helmschale sowie einem Visier, sind an zwei entgegengesetzten Seiten des Schutzhelmes jeweils ein Kopplungsabschnitt der Helmschale und ein Kopplungsabschnitt des Visiers ausgebildet, von denen einer als Kopplungsaufnahme und der andere als Kopplungsfortsatz ausgebildet ist und zwar derart, dass der Kopplungsfortsatz in die Kopplungsaufnahme eingreifen kann, um das Visier mit der Helmschale zu koppeln. Der Kopplungsfortsatz ist dabei in der Kopplungsaufnahme zwischen einer Ausgangsposition und einer Vorschubposition entlang einer Verschiebestrecke verschiebbar und zwischen der Vorschubposition und einer Aufklappposition um eine Schwenkachse schwenkbar.

## Beschreibung

Die Erfindung betrifft einen Schutzhelm, insbesondere einen Sporthelm, mit einer stoßabsorbierenden Helmschale sowie einem Visier.

Schutzhelme können bei verschiedenen Arten von Aktivitäten zweckmäßig sein, um den Kopf des jeweiligen Helmträgers zu schützen. Dabei kann es sich insbesondere um sportliche Aktivitäten, wie beispielsweise Fahrradfahren, handeln. In diesem Fall kann der Schutzhelm als Sporthelm ausgebildet sein.

Ein Schutzhelm umfasst in der Regel eine Helmschale, die auf den Kopf des jeweiligen Helmträgers aufgesetzt werden kann und stoßabsorbierend ist, so dass ein gegen den Kopf erfolgender Stoß von dem Schutzhelm aufgefangen und zumindest abgeschwächt werden kann. Auf diese Weise kann die Gefahr einer durch einen solchen Stoß verursachten Verletzung des Helmträgers reduziert werden. Beispielweise kann die Helmschale eine dünne Außenschale aus einem harten Material sowie einen wesentlich dickeren Helmkörper aufweisen, der ein deformierbares, insbesondere geschäumtes, Material umfasst oder aus diesem gebildet ist. Dabei ist es zweckmäßig, wenn die Helmschale bei aufgesetztem Schutzhelm weite Teile des Kopfes abdeckt, insbesondere sich zumindest von der Stirn bis ins Genick und seitlich bis zu den Schläfen und um die Ohren herum erstreckt.

Zusätzlich kann es zweckmäßig sein, auch den Bereich der Augen zu schützen, sei es mechanisch vor Stößen oder dem Eindringen von Objekten, wie Staub oder Pollen, oder optisch vor übermäßiger Lichteinstrahlung. Diese Funktion kann in der Regel nicht von der Helmschale selbst erfüllt werden, da diese üblicherweise opak ist und außerdem zu dick wäre. Allerdings kann ein Schutzhelm zum Schutz des Augenbereichs ein Visier aufweisen, das die Augen sowohl mechanisch als auch, beispielsweise durch eine entsprechende Tönung, optisch schützen kann.

Grundsätzlich kann ein solches Visier starr an der Helmschale befestigt sein. Oftmals ist es jedoch bevorzugt, wenn sich das Visier zwischen einer Geschlossenstellung, in der es bei aufgesetztem Schutzhelm den Augenbereich des Kopfes des jeweiligen Helmträgers abdeckt, und einer Offenstellung, in der es (typischerweise nach oben vor die Stirn oder noch weiter) aufgeklappt ist, verstellen lässt. Zudem kann es vorteilhaft sein, wenn das Visier auf einfache Weise vollständig von der Helmschale gelöst und auf ebenso einfache Weise auch wieder daran befestigt werden kann. Auf diese Weise kann ein Nutzer des Schutzhelms das Visier wahlweise je nach Bedarf an der Helmschale anbringen oder nicht. Dabei sollte jedoch sichergestellt sein, dass die Art der Befestigung des Visiers an der Helmschale derart ist, dass ein unbeabsichtigtes Lösen des Visiers während der jeweiligen Aktivität hinreichend zuverlässig ausgeschlossen werden kann.

Herkömmliche Schutzhelme mit klappbarem Visier weisen typischerweise vergleichsweise große Scharniermechaniken auf. Falls dabei überhaupt vorgesehen ist, dass sich das Visier auf einfache Weise vollständig von der Helmschale lösen lässt, verbleibt nach dem Lösen in der Regel zumindest ein Teil der jeweiligen Scharniermechanik an der Helmschale, wo er dann übermäßigen Platz beansprucht und optisch als störend wahrgenommen wird.

Es ist eine Aufgabe der Erfindung, einen Schutzhelm, insbesondere einen Sporthelm, mit einem Visier bereitzustellen, bei dem das Visier an der Helmschale des Schutzhelms über eine zuverlässige Mechanik befestigt ist, die sich besonders kompakt ausbilden und auf besonders unauffällige Weise in den Schutzhelm integrieren lässt, dabei ein Verstellen des Visiers zwischen einer Offenstellung und einer Geschlossenstellung erlaubt und idealerweise außerdem ermöglicht, das Visier auf besonders einfache Weise je nach Bedarf an der Helmschale des Schutzhelms zu befestigen oder aber vollständig von der Helmschale zu lösen.

Die Aufgabe wird gelöst durch einen Schutzhelm mit den Merkmalen des Anspruchs 1. Vorteilhafte Ausführungsformen der Erfindung ergeben sich aus den abhängigen Ansprüchen, der vorliegenden Beschreibung sowie den Figuren.

Der erfindungsgemäße Schutzhelm umfasst eine stoßabsorbierende Helmschale sowie ein Visier. Die Helmschale kann insbesondere dazu ausgebildet sein, auf den Kopf eines Helmträgers aufgesetzt werden. Dazu kann die Helmschale zumindest näherungsweise eine halbkugelschalenartige Form aufweisen. Das Visier kann insbesondere dazu ausgebildet sein, vor den Augen des jeweiligen Helmträgers angeordnet zu werden. Vorzugsweise ist das Visier zumindest teilweise transparent.

Der Schutzhelm kann eine Oberseite sowie eine Vorderseite, eine Rückseite, eine erste Seite und eine zweite Seite aufweisen, die an die Oberseite angrenzen, wobei die Vorderseite und die Rückseite entgegengesetzt zueinander ausgerichtet sind und wobei die erste Seite und die zweite Seite ebenfalls entgegensetzt zueinander ausgerichtet sind und jeweils sowohl an die Vorderseite als auch an die Rückseite angrenzen. Die Vorderseite kann zumindest im Wesentlichen in die Blickrichtung eines jeweiligen Helmträgers weisend ausgerichtet sein und die Stirn des jeweiligen Helmträgers abdecken, wenn der Schutzhelm in dazu vorgesehener Weise auf den Kopf des jeweiligen Helmträgers aufgesetzt ist, während die Rückseite in die entgegengesetzte Richtung weisend ausgerichtet ist und bei aufgesetztem Schutzhelm den Hinterkopf des jeweiligen Helmträgers abdeckt. Die erste Seite und die zweite Seite können jeweils zumindest im Wesentlichen in zu diesen Richtungen senkrechter Richtung weisend ausgerichtet sein. Die Helmschale kann ebenfalls eine Oberseite, eine Vorderseite, eine Rückseite, eine erste Seite und eine zweite Seite aufweisen, die der genannten Oberseite, Vorderseite, Rückseite, ersten Seite und zweiten Seite des Schutzhelmes entsprechen.

Das Visier kann an der ersten Seite und an der zweiten Seite des Schutzhelmes mit der Helmschale verbunden sein. Dabei kann das Visier zumindest im Wesentlichen die Form einer radial nach außen weisenden Oberfläche eines Kugelschnitzes (d.h. eine länglich gebogene Form mit zu den Enden ihrer Längserstreckung hin abnehmender Breite, insbesondere mit spitz zulaufenden Enden) aufweisen. In einer Geschlossenstellung kann sich das Visier dabei insbesondere entlang der genannten Vorderseite des Schutzhelmes von der ersten Seite zur zweiten Seite erstrecken. In einer Offenstellung kann sich das Visier dagegen insbesondere entlang eines Übergangs von der Vorderseite zur Oberseite des Schutzhelmes oder entlang der Oberseite des Schutzhelmes von der ersten Seite der zweiten Seite erstrecken.

Erfindungsgemäß sind an zwei entgegengesetzten Seiten des Schutzhelmes jeweils ein Kopplungsabschnitt der Helmschale und ein Kopplungsabschnitt des Visiers ausgebildet. Bei diesen zwei entgegengesetzten Seiten des Schutzhelmes kann es sich insbesondere um die genannte erste Seite und die genannte zweite Seite des Schutzhelmes handeln, die einer ersten Seite bzw. einer zweiten Seite der Helmschale entsprechen. Durch das Zusammenwirken des jeweiligen Kopplungsabschnitts der Helmschale mit dem jeweiligen Kopplungsabschnitt des Visiers an den beiden Seiten des Schutzhelmes kann das Visier mit der Helmschale verbunden sein.

Dabei ist an beiden genannten Seiten des Schutzhelmes jeweils von den beiden an der jeweiligen Seite ausgebildeten Kopplungsabschnitten (nämlich dem an der jeweiligen Seite ausgebildeten Kopplungsabschnitt der Helmschale und dem an der jeweiligen Seite ausgebildeten Kopplungsabschnitt des Visiers) einer als Kopplungsaufnahme und der andere als Kopplungsfortsatz ausgebildet. Vorzugsweise sind die an der Helmschale ausgebildeten Kopplungsabschnitte jeweils als Kopplungsaufnahme ausgebildet, während die an dem Visier ausgebildeten Kopplungsabschnitte als Kopplungsfortsatz ausgebildet sind. Sofern eine jeweilige Kopplungsaufnahme an der Helmschale ausgebildet ist, ist diese vorzugsweise als Ausnehmung in dem genannten Helmkörper der Helmschale ausgebildet.

Die Kopplungsaufnahme und der Kopplungsfortsatz sowie deren Zusammenwirken werden im Folgenden näher beschrieben. Dabei ist überwiegend jeweils von einer Kopplungsaufnahme bzw. einem Kopplungsfortsatz im Singular die Rede. Da an beiden genannten Seiten des Schutzhelmes jeweils ein als Kopplungsaufnahme ausgebildeter Kopplungsabschnitt (der Helmschale oder des Visiers) sowie ein als Kopplungsfortsatz ausgebildeter Kopplungsabschnitt (des jeweils anderen Teils) vorgesehen sind, ist die Beschreibung trotz des Singulars nicht auf die eine Kopplungsaufnahme bzw. den einen Kopplungsfortsatz (auf einer der beiden Seiten) beschränkt zu verstehen; sondern es können jeweils sowohl die Kopplungsaufnahme auf der einen Seite als auch die Kopplungsaufnahme auf der anderen Seite des Schutzhelmes bzw. sowohl der Kopplungsfortsatz auf der einen Seite als auch der Kopplungsfortsatz auf der anderen Seite des Schutzhelmes auf die jeweils beschriebene Weise ausgebildet sein. Vorzugweise sind die beiden Kopplungsabschnitte auf der einen Seite des Schutzhelmes und die beiden Kopplungsabschnitte auf der anderen Seite des Schutzhelmes auf zueinander entsprechende Weise, insbesondere spiegelbildlich zueinander, ausgebildet.

Erfindungsgemäß sind die Kopplungsaufnahme und der Kopplungsfortsatz derart ausgebildet, dass der Kopplungsfortsatz in die Kopplungsaufnahme eingreifen kann, um das Visier mit der Helmschale zu koppeln. Mit anderen Worten erfolgt die Kopplung zwischen dem Visier und der Helmschale zumindest unter anderem dadurch, dass der Kopplungsfortsatz in die Kopplungsaufnahme eingreift. Die Eingriffsrichtung, d. h. die Richtung des Eingreifens des Kopplungsfortsatzes in die Kopplungsaufnahme, kann dabei insbesondere radial (nach innen oder nach au-ßen) bezüglich eines Mittelpunkts der Helmschale (insbesondere eines Mittelpunkts der genannten halbkugelschalenartigen Form der Helmschale) ausgerichtet sein. Das genannte Koppeln kann zum einen der Befestigung des Visiers an der Helmschale dienen, aber auch einer beweglichen Lagerung des Visiers an der Helmschale. Zudem ist die Kopplung vorzugsweise reversibel (d. h. beabsichtigt lösbar), damit das Visier wahlweise an der Helmschale angeordnet oder aber, wenn es nicht gebraucht wird, weggelassen werden kann.

Des Weiteren ist erfindungsgemäß vorgesehen, dass der Kopplungsfortsatz in der Kopplungsaufnahme, d. h. wenn er in die Kopplungsaufnahme eingreift, zwischen einer Ausgangsposition und einer Vorschubposition entlang einer Verschiebestrecke verschiebbar und zwischen der Vorschubposition und einer Aufklappposition um eine Schwenkachse schwenkbar ist. Mit anderen Worten können die Kopplungsaufnahme und der Kopplungsfortsatz derart relativ zueinander ausgebildet sein, dass der Kopplungsfortsatz in der Kopplungsaufnahme zwischen der Ausgangsposition und der Vorschubposition entlang der Verschiebestrecke verschiebbar und zwischen der Vorschubposition und der Aufklappposition um eine Schwenkachse schwenkbar ist.

Der Kopplungsfortsatz kann also die genannte Ausgangsposition, die genannte Vorschubposition und die genannte Aufklappposition einnehmen, wobei sich der Kopplungsfortsatz in jeder dieser Positionen in der Kopplungsaufnahme befindet. Außerdem kann der Kopplungsfortsatz aus der Ausgangsposition entlang der genannten Verschiebestrecke in die Vorschubposition verschoben werden und aus der Vorschubposition um die genannte Schwenkachse in die Aufklappposition geschwenkt werden sowie umgekehrt aus der Aufklappposition um die genannte Schwenkachse in die Vorschubposition geschwenkt werden und aus der Vorschubposition entlang der genannten Verschiebestrecke in die Ausgangsposition verschoben werden. Auch während dieser Bewegungen (Verschieben und Schwenken) befindet sich der Kopplungsfortsatz jeweils in der Kopplungsaufnahme.

Insbesondere können die Kopplungsaufnahme und der Kopplungsfortsatz derart ausgebildet sein, dass eine Bewegung des Kopplungsfortsatzes aus der Ausgangsposition in die Aufklappposition oder aus der Aufklappposition in die Ausgangsposition zwangsläufig über die Vorschubposition führt. Ferner ist es vorteilhaft, wenn die Ausgangsposition und die Aufklappposition entgegengesetzte Endpositionen der Bewegung des Kopplungsfortsatzes innerhalb der Kopplungsaufnahme sind, über die hinaus (in Richtung von der jeweils entgegensetzten Endposition weg) der Kopplungsfortsatz innerhalb der Kopplungsaufnahme nicht bewegt werden kann. Aus der Ausgangsposition kann der Kopplungsfortsatz jedoch außerdem (entgegen der genannten Eingriffsrichtung) aus der Kopplungsaufnahme entnehmbar sein.

Die Bezeichnungen der drei genannten Positionen (Ausgangsposition, Vorschubposition und Aufklappposition) dienen primär der begrifflichen Unterscheidung dieser Positionen voneinander. Grundsätzlich könnten diese Positionen auch als erste Position, zweite Position bzw. dritte Position bezeichnet werden. Der Kopplungsfortsatz ist zweckmäßigerweise starr mit dem Visier bzw. mit der Helmschale (je nachdem, ob er an dem Visier oder an der Helmschale ausgebildet ist) verbunden, so dass die genannten Positionen entsprechenden Position des Visiers relativ zu der Helmschale entsprechen. Insbesondere kann die Ausgangsposition des Kopplungsfortsatzes einer Geschlossenstellung des Visiers entsprechen und die Aufklappposition des Kopplungsfortsatzes einer Offenstellung des Visiers entsprechen.

Bei einigen Ausführungsformen können der jeweilige Kopplungsfortsatz und die zugeordnete Kopplungsaufnahme dazu ausgebildet sein, dass der Kopplungsfortsatz lediglich in der Vorschubposition schwenkbar ist, nicht jedoch in der Ausgangsposition oder entlang der Verschiebestrecke. Dies vereinfacht für den Benutzer eine intuitive, haptisch geführte Bedienung des Visiers.

Bei einigen Ausführungsformen können der jeweilige Kopplungsfortsatz und die zugeordnete Kopplungsaufnahme dazu ausgebildet sein, dass der Kopplungsfortsatz bezogen auf eine Ebene, die sich quer zu der genannten Eingriffsrichtung erstreckt und die Verschiebestrecke umfasst, ausgehend von der Ausgangsposition lediglich entlang der Verschiebestrecke bewegbar ist, nicht jedoch in andere Richtungen. Auch dies vereinfacht für den Benutzer eine intuitive, haptisch geführte Bedienung des Visiers.

Die genannte Verschiebestrecke, entlang welcher der Kopplungsfortsatz zwischen der Ausgangsposition und der Vorschubposition verschiebbar ist, ist vorzugsweise zumindest im Wesentlichen geradlinig. Mit anderen Worten handelt es sich bei einem Verschieben des Kopplungsfortsatzes in der Kopplungsaufnahme entlang der Verschiebestrecke zumindest im Wesentlichen um eine reine Translation. Dabei kann es zweckmäßig sein, wenn die Verschiebestrecke ausgehend von der Ausgangsposition zunächst einen exakt geradlinigen Abschnitt umfasst (reine Translation), an den sich ein demgegenüber geringfügig abgewinkelter und/oder geringfügig gebogener Vorschwenkabschnitt anschließt, der sich bis zur Vorschubposition erstreckt, oder wenn die Verschiebestrecke insgesamt als ein geringfügig gebogener Vorschwenkabschnitt ausgebildet ist. Ein solcher Vorschwenkabschnitt kann (als eine Art Anlaufschräge) den Übergang zwischen einem Verschieben des Kopplungsfortsatzes entlang der Verschiebestrecke und einem Schwenken des Kopplungsvorsatzes um die Schwenkachse im Bereich der Vorschubposition unterstützen und somit erleichtern.

Der aus einem solchen Vorschwenkabschnitt resultierende Rotationsanteil an der Bewegung des Kopplungsfortsatzes entlang der Verschiebestrecke ist dabei vorzugweise im Vergleich zum Translationsanteil relativ gering. Beispielweise kann die Bewegung des Kopplungsfortsatzes entlang der Verschiebestrecke einen Rotationsanteil von höchstens 10°, vorzugsweise höchstens 5° aufweisen. Dagegen weist die Bewegung des Kopplungsfortsatzes entlang der Verschiebestrecke vorzugsweise einen Translationsanteil von zumindest 10 mm, vorzugweise von zumindest 12 mm auf.

Des Weiteren ist es bevorzugt, wenn die Schwenkachse, um die der Kopplungsfortsatz zwischen der Vorschubposition und der Aufklappposition schwenkbar ist, zumindest im Wesentlichen senkrecht zur Verschiebestrecke ausgerichtet ist. Falls die Verschiebestrecke nicht exakt geradlinig ist, ist die Schwenkachse vorzugsweise senkrecht zu einer Ebene ausgerichtet, innerhalb welcher die Verschiebestrecke verläuft. Grundsätzlich unabhängig davon ist es bevorzugt, wenn die Schwenkachse zu der genannten Eingriffsrichtung, in der der Kopplungsfortsatz in die Kopplungsaufnahme eingreift, parallel ausgerichtet ist. Insofern ist die Verschiebestrecke bzw. die genannte Ebene, innerhalb welcher sie verläuft, vorzugsweise senkrecht zur Eingriffsrichtung ausgerichtet.

Bei dem Schwenken des Kopplungsfortsatzes zwischen der Vorschubposition und der Aufklappposition handelt es sich vorzugsweise um eine reine Rotation. Allerdings kann diese Schwenkbewegung grundsätzlich auch einen vergleichsweise geringen Translationsanteil aufweisen. Vorzugsweise beträgt dieser Translationsanteil höchstens 2 mm, insbesondere höchstens 1 mm. Der Rotationsanteil der Schwenkbewegung zwischen der Vorschubposition und der Aufklappposition beträgt dagegen vorzugsweise mindestens 25°, insbesondere mindestens 30°.

Durch die Kopplung des Visiers mit der Helmschale über das beschriebene beidseitig des Schutzhelmes erfolgende Eingreifen eines jeweiligen Kopplungsfortsatzes in eine jeweilige Kopplungsaufnahme kann das Visier zuverlässig an der Helmschale befestigt sein und dabei zwischen verschiedenen Stellungen (insbesondere zwischen der genannten Geschlossenstellung und der genannten Offenstellung) verstellbar sein. Zudem kann diese Kopplung besonders kompakt und weitgehend unauffällig in die Helmschale integriert ausgebildet sein. Das gilt insbesondere, wenn die Kopplungsaufnahmen jeweils in der Helmschale ausgebildet sind, beispielsweise als eine Ausnehmung im Helmkörper, in die ein jeweiliger an dem Visier ausgebildeter Kopplungsfortsatz eingreift.

Gemäß einer vorteilhaften Ausführungsform umfasst die Kopplungsaufnahme einen Verschiebeabschnitt, der zumindest im Wesentlichen die Form eines Langlochs aufweist, und einen Schwenkabschnitt, der zumindest im Wesentlichen die Form eines Kreissektors aufweist. Der Verschiebeabschnitt kann in den Schwenkabschnitt münden. Der Verschiebeabschnitt und der Schwenkabschnitt können dabei auch überlappen. Vorzugsweise bilden der Verschiebeabschnitt und der Schwenkabschnitt gemeinsam die Kopplungsaufnahme. Bevorzugt befindet sich der Kopplungsfortsatz während eines Verschiebens zwischen der Ausgangsposition und der Vorschubposition entlang der Verschiebestrecke zumindest im Wesentlichen innerhalb des Verschiebeabschnitts. Während eines Schwenkens zwischen der Vorschubposition und der Aufklappposition um die Schwenkachse befindet sich der Kopplungsfortsatz dagegen bevorzugt zumindest im Wesentlichen innerhalb des Schwenkabschnitts.

Die beschriebenen Formen des Verschiebeabschnitts und des Schwenkabschnitts können jeweils insbesondere auf einen zu der genannten Eingriffsrichtung und/ oder zu der Schwenkachse senkrechten Querschnitt bezogen sein. Parallel zur Eingriffsrichtung bzw. zur Schwenkachse ist dieser Querschnitt des Verschiebeabschnitts bzw. des Schwenkabschnitts vorzugsweise zumindest im Wesentlichen konstant.

Der Verschiebeabschnitt kann insbesondere insofern die Form eines Langlochs aufweisen, als er eine längliche Form aufweist, die sich entlang der Verschiebestrecke erstreckt und dabei senkrecht zum Verlauf der Verschiebestrecke (sowie senkrecht zur Eingriffsrichtung und/oder zur Schwenkachse) eine zumindest im Wesentlichen konstante Breite aufweist, d. h. durch zwei Ränder mit konstantem Abstand begrenzt wird.

Der Schwenkabschnitt kann insbesondere insofern die Form eines Kreissektors aufweisen, als diese Form in zur Schwenkachse radialer Richtung durch einen Kreisbogen und in Umlaufrichtung um die Schwenkachse (d. h. in die beiden Schwenkrichtungen in die der Kopplungsfortsatz zwischen der Vorschubposition und der Aufklappposition schwenkbar ist) jeweils durch einen Kreisradius begrenzt wird. Einer der beiden Kreisradien ist vorzugsweise zumindest im Wesentlichen in Verlängerung eines der genannten Ränder des Verschiebeabschnitts, insbesondere parallel zu diesem Rand, ausgerichtet. Allerdings kann er auch als Anlaufschräge ausgebildet sein, indem er geringfügig gebogen oder relativ zu dem genannten Rand geringfügig abgewinkelt ist. Insbesondere kann sich daraus der genannte Vorschwenkabschnitt der Verschiebestrecke ergeben.

Je nach Form des Kopplungsfortsatzes und/oder nach Art der Verbindung des Kopplungsfortsatzes mit dem übrigen Visier bzw. mit der übrigen Helmschale kann die Kopplungsaufnahme auch zwei Schwenkabschnitte umfassen, die jeweils zumindest im Wesentlichen die Form eines Kreissektors aufweisen. Bezüglich der Schwenkachse können diese zwei Schwenkabschnitte dann insbesondere diametral entgegengesetzt zueinander angeordnet sein, so dass sie gemeinsam eine Schmetterlingsform bilden.

Gemäß einer weiteren vorteilhaften Ausführungsform umfasst der Kopplungsfortsatz einen Sockelabschnitt mit einer länglichen Form, der sich entlang einer Längsachse erstreckt und der in der Ausgangsposition, in der Vorschubposition und in der Aufklappposition vollständig in der Kopplungsaufnahme aufgenommen ist, sowie einen Verbindungsabschnitt, der sich entlang einer zur Längsachse quer, insbesondere senkrecht, ausgerichteten Querachse erstreckt und den Sockelabschnitt mit dem übrigen Visier bzw. mit der übrigen Helmschale (je nachdem, ob der Kopplungsfortsatz an dem Visier oder an der Helmschale ausgebildet ist) verbindet. Vorzugsweise bilden der Sockelabschnitt und der Verbindungsabschnitt gemeinsam den Kopplungsfortsatz.

Die beschriebene Form des Sockelabschnitts kann insbesondere auf einen zur Eingriffsrichtung und/oder zur Schwenkachse senkrechten Querschnitt bezogen sein. Parallel zur Eingriffsrichtung bzw. zur Schwenkachse ist der Querschnitt des Sockelabschnitts vorzugsweise zumindest im Wesentlichen konstant.

Der Sockelabschnitt kann insbesondere insofern eine längliche Form umfassen, als er (zumindest im Querschnitt) entlang der genannten Längsachse seine größte Erstreckung aufweist und sich in dazu senkrechte Richtung(en) deutlich weniger weit, insbesondere höchstens halb so weit, erstreckt. Beispielsweise kann die längliche Form im Querschnitt durch zwei diametral entgegengesetzte voneinander beabstandete Halbkreise gebildet werden, die an ihren Enden über zwei zueinander parallele Linien miteinander verbunden sind.

Der Verbindungsabschnitt kann insbesondere eine Zylinderform aufweisen. Die genannte Querachse, entlang welcher der Verbindungsabschnitt den Sockelabschnitt mit dem übrigen Visier bzw. mit der übrigen Helmschale verbindet, kann dann der Zylinderachse dieser Zylinderform entsprechen. Der Kopplungsfortsatz kann insbesondere parallel zu dieser Querachse in die Kopplungsaufnahme eingreifen. Insofern ist die Querachse dann parallel zu der genannten Eingriffsrichtung. Dabei kann der Kopplungsfortsatz in die Kopplungsaufnahme so weit eingreifen, dass der Sockelabschnitt vollständig in der Kopplungsaufnahme aufgenommen ist, wohingegen sich der Verbindungsabschnitt (in allen drei genannten Positionen des Kopplungsfortsatzes sowie vorzugsweise auch in sämtlichen dazwischenliegenden Positionen) vom Sockelabschnitt aus entgegen der Eingriffsrichtung aus der Kopplungsaufnahme heraus zum übrigen Visier bzw. zur übrigen Helmschale erstreckt.

Vorzugsweise ist der Sockelabschnitt dabei starr mit dem Verbindungsabschnitt verbunden und der Verbindungsabschnitt starr mit dem übrigen Visier bzw. der übrigen Helmschale verbunden, so dass insgesamt der gesamte Kopplungsfortsatz an dem Visier bzw. an der Helmschale starr ausgebildet ist. Dadurch entspricht ein Verschieben oder Schwenken des Kopplungsfortsatzes zwangsläufig einem entsprechenden Verschieben bzw. Schwenken des Visiers bzw. der Helmschale, an dem/der der Kopplungsfortsatz ausgebildet ist.

Die beschriebenen Ausbildungen der Kopplungsaufnahme und des Kopplungsfortsatzes ermöglichen es insbesondere, dass in der Ausgangsposition des Kopplungsfortsatzes dessen Sockelabschnitt mit seiner Längsachse parallel zur Verschiebestrecke ausgerichtet an einem vom Schwenkabschnitt beabstandeten Ende des Verschiebeabschnitts der Kopplungsaufnahme in der Kopplungsaufnahme angeordnet ist. Er kann dann innerhalb des Verschiebeabschnitts zumindest im Wesentlichen parallel zu seiner Längsachse entlang der Verschiebestrecke bis zu einem entgegengesetzten Ende des Verschiebeabschnitts, mit dem der Verschiebeabschnitt in den Schwenkabschnitt mündet oder mit diesem überlappt, verschoben werden, so dass der Kopplungsfortsatz dann die Vorschubposition einnimmt.

In der Vorschubposition kann der Sockelabschnitt des Kopplungsfortsatzes dann innerhalb des Schwenkabschnitts der Kopplungsaufnahme geschwenkt werden. Vorzugsweise wird der Kopplungsfortsatz dabei um die Querachse seines Verbindungsabschnitts geschwenkt, die insofern mit der Schwenkachse zusammenfällt. Dabei kann der Sockelabschnitt die Kreissektorform des Schwenkabschnitts von dem einen genannten Kreisradius zu dem anderen genannten Kreisradius, die den Schwenkabschnitt in Umlaufrichtung um die Schwenkachse begrenzen können, durchlaufen. Letztlich nimmt der Kopplungsfortsatz nach dem Schwenken dann die Aufklappposition ein. In umgekehrter Richtung kann die Bewegung von der Aufklappposition über die Vorschubposition in die Ausgangsposition gerade umgekehrt ablaufen.

Aufgrund der beschriebenen Ausbildung des Sockelabschnitts und des Verbindungsabschnitts kann der Sockelabschnitt bezüglich der genannten Querachse, entlang welcher sich der Verbindungsabschnitt erstreckt, insbesondere radial ausgerichtet sein. Je nachdem, wo entlang der Längsachse des Sockelabschnitts der Verbindungsabschnitt mit dem Sockelabschnitt verbunden ist, kann sich der Sockelabschnitt von der Querachse des Verbindungsabschnitts aus in nur eine radiale Richtung oder aber in zwei entgegengesetzte radiale Richtungen erstrecken.

Beispielweise kann der Verbindungsabschnitt an einem Ende der Erstreckung des Sockelabschnitts entlang der Längsachse (d. h. an einem von zwei Enden, zwischen denen sich der Sockelabschnitt entlang der Längsachse erstreckt) mit dem Sockelabschnitt verbunden sein. Vorzugsweise ist der Verbindungsabschnitt dabei nur an diesem einen Ende mit dem Sockelabschnitt verbunden. Alternativ dazu kann der Verbindungsabschnitt aber auch in einem mittleren Bereich der Erstreckung des Sockelabschnitts entlang der Längsachse, insbesondere exakt in der Mitte dieser Längserstreckung, mit dem Sockelabschnitt verbunden sein.

Gemäß einer weiteren vorteilhaften Ausführungsform ist der Kopplungsfortsatz beim Verschieben zwischen der Ausgangsposition und der Vorschubposition und/ oder beim Schwenken zwischen der Vorschubposition und der Aufklappposition zwangsgeführt. Das kann insbesondere umfassen, dass die jeweilige Bewegung des Kopplungsfortsatzes innerhalb der Kopplungsaufnahme nur genau einen Freiheitsgrad aufweist. Die Einschränkung auf den einen Freiheitsgrad kann sich dabei insbesondere aus einem unmittelbaren, vorzugsweise zumindest teilweise führenden, Zusammenwirken des Kopplungsfortsatzes und der Kopplungsaufnahme miteinander ergeben.

Gemäß einer weiteren vorteilhaften Ausführungsform ist an der Kopplungsaufnahme eine Führungsstruktur ausgebildet, die mit einer an dem Kopplungsfortsatz ausgebildeten Führungsstruktur derart zusammenwirkt, dass der Kopplungsfortsatz beim Verschieben zwischen der Ausgangsposition und der Vorschubposition entlang der Verschiebestrecke geführt wird. Das Führen des Kopplungsfortsatzes kann sich insbesondere daraus ergeben, dass die genannten Führungsstrukturen aneinander entlanggleiten und dadurch eine Bewegung des Kopplungsfortsatzes entlang einer bestimmten Strecke, insbesondere der Verschiebestrecke, geleitet wird.

Für eine zuverlässige Führung können die Führungsstrukturen dabei vorteilhafterweise ineinandergreifen. Beispielsweise können die Führungsstrukturen nach Art einer Nut-Feder-Verbindung ausgebildet sein, also einerseits zumindest eine Nut aufweisen und andererseits zumindest eine Feder (im Sinne eines Stegs) aufweisen, die in die Nut eingreift. Die Führung ergibt sich dann insbesondere aus dem Gleiten der Feder in der Nut.

Die Führungsstruktur des Kopplungsfortsatzes kann insbesondere an dem genannten Sockelabschnitt des Kopplungsfortsatzes, vorzugsweise an zumindest einem in zu der Längsachse und zu der Querachse senkrechte Richtung weisenden Rand des Sockelabschnitts, ausgebildet sein. Bevorzugt ist an zwei entgegengesetzten solchen Rändern des Sockelabschnitts ein jeweiliger Teil der Führungsstruktur ausgebildet.

Die Führungsstruktur der Kopplungsaufnahme kann insbesondere an dem Verschiebeabschnitt der Kopplungsaufnahme, vorzugsweise an zumindest einem die Kopplungsaufnahme quer zur Eingriffsrichtung begrenzenden Rand des Verschiebeabschnitts, ausgebildet sein. Bevorzugt ist an zwei entgegengesetzten solchen Rändern des Verschiebeabschnitts ein jeweiliger Teil der Führungsstruktur ausgebildet.

Des Weiteren ist es bevorzugt, wenn die an der Kopplungsaufnahme ausgebildete Führungsstruktur und die an dem Kopplungsfortsatz ausgebildete Führungsstruktur jeweils einen Führungssteg aufweisen und der Führungssteg des Kopplungsfortsatzes den Führungssteg der Kopplungsaufnahme derart hintergreift, dass der Kopplungsfortsatz entlang der Verschiebestrecke formschlüssig gegen ein Verlassen der Kopplungsaufnahme (d. h. gegen eine Bewegung, insbesondere entgegen der Eingriffsrichtung, aus der Kopplungsaufnahme heraus) gesichert ist. Der Führungssteg kann dabei jeweils durch einen frei vorstehenden Steg oder auch durch den Rand einer Nut gebildet sein. Wichtig für die formschlüssigen Sicherung ist dabei insbesondere, dass durch den an der Kopplungsaufnahme ausgebildeten Führungssteg eine Hinterschneidung gebildet wird, die von dem an dem Kopplungsfortsatz ausgebildeten Führungssteg hintergriffen werden kann.

Gemäß einer weiteren vorteilhaften Ausführungsform kann der Kopplungsfortsatz in der Kopplungsaufnahme durch einen Formschluss (insbesondere durch ein Hintergreifen von Teilen der Kopplungsaufnahme) gegen ein Verlassen der Kopplungsaufnahme entgegen der genannten Eingriffsrichtung gesichert sein, sobald der Kopplungsfortsatz ausgehend von der Ausgangsposition entlang der Verschiebestrecke bewegt worden ist. Hierdurch kann das Visier besonders zuverlässig an der Helmschale gesichert sein, sobald der Benutzer das Visier aus der Ausgangsposition herausbewegt hat. In der Ausgangsposition kann das Visier bei einigen Ausführungsformen durch andere Maßnahmen gegen ein unbeabsichtigtes Verlassen der Kopplungsaufnahme entgegen der Eingriffsrichtung gesichert sein, beispielsweise durch einen magnetischen Kraftschluss oder durch einen mechanischen Kraftschluss (insbesondere durch Verrasten).

Insbesondere kann der Kopplungsfortsatz in der Vorschubposition, in der Aufklappposition, entlang der Verschiebestrecke sowie zwischen der Vorschubposition und der Aufklappposition (mit letzterem sind alle Positionen gemeint, die beim Schwenken zwischen der Vorschubposition und der Aufklappposition durchlaufen werden) formschlüssig gegen ein Verlassen der Kopplungsaufnahme gesichert sein. Insbesondere ist es bevorzugt, wenn der Kopplungsfortsatz zumindest im Wesentlichen ausschließlich in der Ausgangsposition die Kopplungsaufnahme verlassen kann. In allen anderen Positionen innerhalb der Kopplungsaufnahme ist der Kopplungsfortsatz vorzugsweise formschlüssig gegen ein Verlassen der Kopplungsaufnahme gesichert.

Entlang der Verschiebestrecke kann der Kopplungsfortsatz beispielsweise durch die genannten Führungsstege formschlüssig in der Kopplungsaufnahme gesichert sein. Zusätzlich und/oder in den übrigen Positionen kann der Kopplungsfortsatz beispielsweise durch eine Außenschale der Helmschale oder eine daran angeordnete Abdeckschale, die sich zumindest teilweise über die Kopplungsaufnahme erstreckt, formschlüssig in der Kopplungsaufnahme gesichert sein, beispielsweise indem zumindest ein Teil des genannten Sockelabschnitts einen Rand dieser Außenschale bzw. Abdeckschale hintergreift.

Um ein einfaches Lösen des Visiers von der Helmschale zu ermöglichen, ist es vorteilhaft, wenn der Kopplungsfortsatz in der Ausgangsposition die Kopplungsaufnahme grundsätzlich verlassen kann, insbesondere indem er in der Ausgangsposition einfach entgegen der Eingriffsrichtung aus der Kopplungsaufnahme gezogen werden kann. Umgekehrt ist es zweckmäßig, wenn für ein einfaches Anbringen des Visiers an der Helmschale der Kopplungsfortsatz einfach in Eingriffsrichtung in die Kopplungsaufnahme eingesetzt werden kann, so dass er dann die Ausgangsposition einnimmt. Dabei ist es für eine zuverlässige Kopplung des Visiers mit der Helmschale wichtig, dass der Kopplungsfortsatz auch in der Ausgangsposition in der Kopplungsaufnahme gesichert ist, so dass er sie möglichst nur bei einem beabsichtigten Lösen verlässt.

Beispielsweise kann der Kopplungsfortsatz aufgrund einer Eigenelastizität des Visiers in die Kopplungsaufnahme vorgespannt sein. Eine solche Eigenelastizität kann beispielsweise die genannten Enden des Visiers von außen gegen die Helmschale vorspannen. In der Ausgangsposition kann der Kopplungsfortsatz dann aufgrund der Vorspannung in der Kopplungsaufnahme gesichert sein, dabei aber gleichwohl einfach, insbesondere durch einfaches entgegengerichtetes Ziehen zur Überwindung der Vorspannung, aus der Kopplungsaufnahme entnommen werden.

Für eine besonders zuverlässige Kopplung des Visiers mit der Helmschale kann der Kopplungsfortsatz in der Ausgangsposition, alternativ zu der genannten Vorspannung oder (vorzugsweise) zusätzlich dazu, auf magnetische Weise in der Kopplungsaufnahme gesichert sein.

Gemäß einer weiteren vorteilhaften Ausführungsform weisen die Kopplungsaufnahme und der Kopplungsfortsatz jeweils ein Magnetelement auf, wobei die Magnetelemente, nämlich das Magnetelement der Kopplungsaufnahme und das Magnetelement des Kopplungsfortsatzes, derart angeordnet sind, dass der Kopplungsfortsatz durch magnetischen Kraftschluss (d. h. die zwischen den beiden Magnetelementen wirkende Anziehung) in der Ausgangsposition gehalten wird. Mit anderen Worten muss dieser magnetischen Kraftschluss überwunden werden, um den Kopplungsfortsatz aus der Ausgangsposition wegzubewegen, insbesondere so weit wegzubewegen, dass er durch die magnetische Anziehung nicht wieder in die Ausgangsposition zurückgezogen wird.

Der magnetischen Kraftschluss muss insbesondere überwunden werden, um den Kopplungsfortsatz entgegen der Eingriffsrichtung aus der Kopplungsaufnahme zu entnehmen (um das Visier von der Helmschale zu lösen). Es kann aber auch erforderlich sein, den magnetischen Kraftschluss zu überwinden, um den Kopplungsfortsatz aus der Ausgangsposition in die Vorschubposition zu verschieben. Die genannten Magnetelemente können dabei dazu ausgebildet (insbesondere derart relativ zueinander angeordnet) sein, dass entgegen der Eingriffsrichtung eine größere Kraft für das Überwinden des magnetischen Kraftschlusses aufgebracht werden muss als in Richtung entlang der Verschiebestrecke zur Vorschubposition hin.

Beispielsweise kann der genannte Sockelabschnitt des Kopplungsfortsatzes oder auch der Kopplungsfortsatz insgesamt ferromagnetisch sein und somit das Magnetelement bilden. Alternativ dazu kann in oder an dem Kopplungsfortsatz, insbesondere in oder an dessen Sockelabschnitt, ein ferromagnetisches Element oder auch ein Permanentmagnet angeordnet sein. In entsprechender Weise kann an der Kopplungsaufnahme, insbesondere in der Nähe des Bereichs der Kopplungsaufnahme, in dem sich der Kopplungsfortsatz in seiner Ausgangsposition befindet, ein ferromagnetisches Element oder auch ein Permanentmagnet angeordnet sein.

Gemäß einer weiteren vorteilhaften Ausführungsform ist an einem Rand der Kopplungsaufnahme eine Stützmulde ausgebildet, an der sich der Kopplungsfortsatz beim Schwenken zwischen der Vorschubposition und der Aufklappposition abstützt. Die Stützmulde kann insbesondere (auch) in einem an dem genannten Rand ausgebildeten Führungssteg ausgebildet sein. Der Kopplungsfortsatz kann sich beim Schwenken insbesondere mit dem genannten Sockelabschnitt an der Stützmulde abstützen, vorzugsweise mit einem Ende der Erstreckung des Sockelabschnitts entlang dessen Längsachse, insbesondere mit demjenigen Ende, an dem der genannte Verbindungsabschnitt mit dem Sockelabschnitt verbunden ist. Der Kopplungsfortsatz kann dabei insofern an der Stützmulde abgestützt sein, als er beim Schwenken zwischen der Vorschubposition und der Aufklappposition (insbesondere zwangsweise) zumindest teilweise in sie eingreift und an ihr entlanggleitet.

Gemäß einer weiteren vorteilhaften Ausführungsform sind die Kopplungsaufnahme und der Kopplungsfortsatz dazu ausgebildet, dass der Kopplungsfortsatz in der Aufklappposition durch Verrasten fixiert ist. Mit anderen Worten rastet beim Schwenken aus der Vorschubposition in die Aufklappposition der Kopplungsfortsatz in die Aufklappposition ein. Für ein Verlassen der Aufklappposition, d. h. für ein Schwenken aus der Aufklappposition in die Vorschubposition, muss dieser Rastschluss durch eine entsprechende Kraft wieder überwunden werden. Auf diese Weise wird das Visier in seiner Offenstellung gehalten und ist somit dagegen gesichert, sich selbständig zu schließen.

Gemäß einer weiteren vorteilhaften Ausführungsform weist die Kopplungsaufnahme einen rückfedernden Rastvorsprung auf, den der Kopplungsfortsatz beim Schwenken zwischen der Vorschubposition und der Aufklappposition passiert und der von dem Kopplungsfortsatz dabei (beim Passieren) vorübergehend zurückgedrängt wird. Das genannte Verrasten kann gerade durch dieses Zusammenwirken des Kopplungsfortsatzes mit dem Rastvorsprung erfolgen. Insbesondere kann der Rastvorsprung von dem Kopplungsfortsatz nur passiert werden, wenn er dabei von dem Kopplungsfortsatz zurückgedrängt wird. Die für dieses Zurückdrängen erforderliche Kraft entspricht somit der für ein Überwinden des Rastschlusses erforderlichen Kraft.

Der Rastvorsprung kann beispielsweise derart angeordnet sein, dass er von dem Sockelabschnitt des Kopplungsfortsatzes zurückgedrängt werden kann, insbesondere von einem Ende der Erstreckung des Sockelabschnitts entlang der genannten Längsachse, das von der genannten Querachse radial weg weisend ausgerichtet ist. Der Rastvorsprung kann beispielsweise an einem Rand der Kopplungsaufnahme, insbesondere integral in diesem Rand, ausgebildet sein. Vorzugsweise ist der Rastvorsprung in dem genannten Schwenkabschnitt, insbesondere an dem genannten Kreisbogen der Kreissektorform des Schwenkabschnitts, ausgebildet.

Alternativ oder zusätzlich zu einer Fixierung durch Verrasten, kann der Kopplungsfortsatz in der Aufklappposition auch magnetisch gesichert sein. Dazu können die Kopplungsaufnahme und der Kopplungsfortsatz jeweils ein Magnetelement aufweisen, die in entsprechender Weise angeordnet sind.

Gemäß einer weiteren vorteilhaften Ausführungsform weist die Helmschale einen Schirm auf, der, wenn sich die Kopplungsfortsätze in ihrer Ausgangsposition befinden, bezüglich der Schwenkachse im Umlaufrichtung an das Visier angrenzt und radial (d. h. in eine bezüglich der Schwenkachse zumindest im Wesentlichen radiale Richtung und somit quer, jedoch nicht unbedingt exakt senkrecht, zur Umlaufrichtung um die Schwenkachse) über das Visier übersteht.

Wenn sich die Kopplungsfortsätze in ihrer Ausgangsposition befinden, nimmt das Visier seine Geschlossenstellung ein. Dadurch, dass der Schirm bei geschlossenem Visier in der genannten Weise über das Visier übersteht, kann das Visier in der Geschlossenstellung nicht einfach direkt um die Schwenkachse an dem Schirm vorbei aufgeklappt werden (wenn die Kopplungsaufnahmen und Kopplungsfortsätze das grundsätzlich zulassen würden). Sondern es ist erforderlich, dass das Visier zunächst zumindest im Wesentlichen in die Richtung, in die der Schirm vorsteht, versetzt wird, damit es an dem Schirm vorbei aufgeklappt werden kann.

Dementsprechend ist bei dieser Ausführungsform ferner vorgesehen, dass das Visier, wenn sich die Kopplungsfortsätze in der Vorschubposition, der Aufklappposition oder dazwischen befinden (und das Visier somit in der genannten Weise versetzt ist), radial über den Schirm hinaus von der Schwenkachse beabstandet angeordnet ist. Das kann insbesondere umfassen, dass das Visier dabei von jedem Punkt der Schwenkachse in zur Schwenkachse radialer Richtung weiter entfernten ist als der Schirm. In dieser versetzten Stellung kann das Visier dann in Umlaufrichtung um die Schwenkachse an dem Schirm vorbei in seine Offenstellung geschwenkt werden. Auf diese Weise schließen sich die Möglichkeit, an der Helmschale wahlweise ein Visier anzubringen, und das Vorsehen eines Schirms an der Helmschale trotz einer vergleichsweise eng anliegenden Anordnung des Visiers an der Helmschale nicht gegenseitig aus.

Die Erfindung wird nachfolgend lediglich beispielhaft unter Bezugnahme auf die Figuren weiter erläutert.
- Fig. 1 bis 4: zeigen eine Ausführungsform eines erfindungsgemäßen Schutzhelmes in vier verschiedenen Stellungen eines Visiers des Schutzhelmes.
- Fig. 5 bis 9: zeigen Detaildarstellungen eines Kopplungsabschnitts einer Helmschale des Schutzhelmes sowie eines damit zusammenwirkenden Kopplungsabschnitts des Visiers.

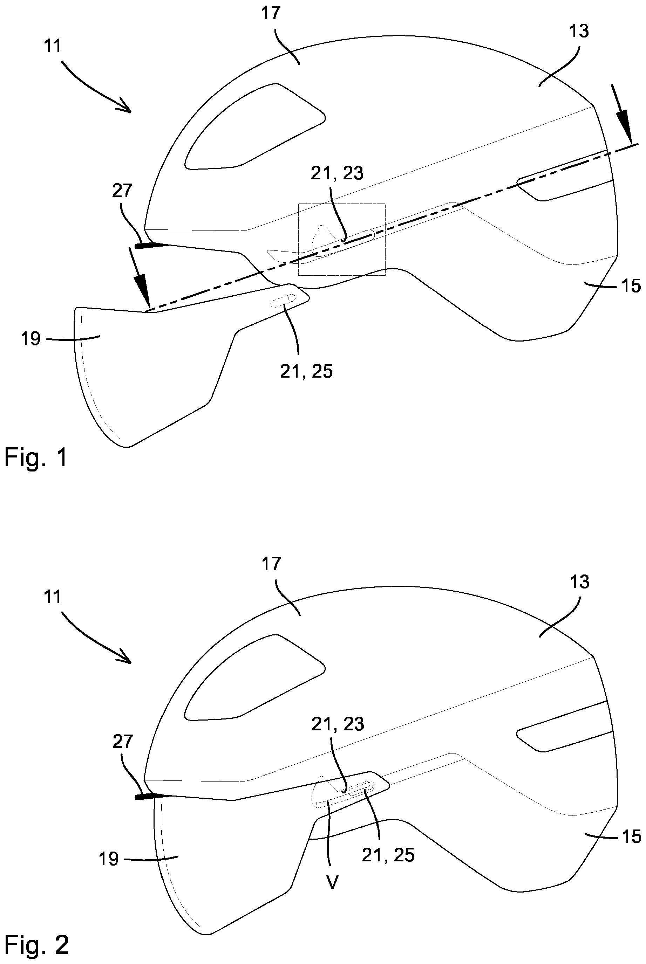

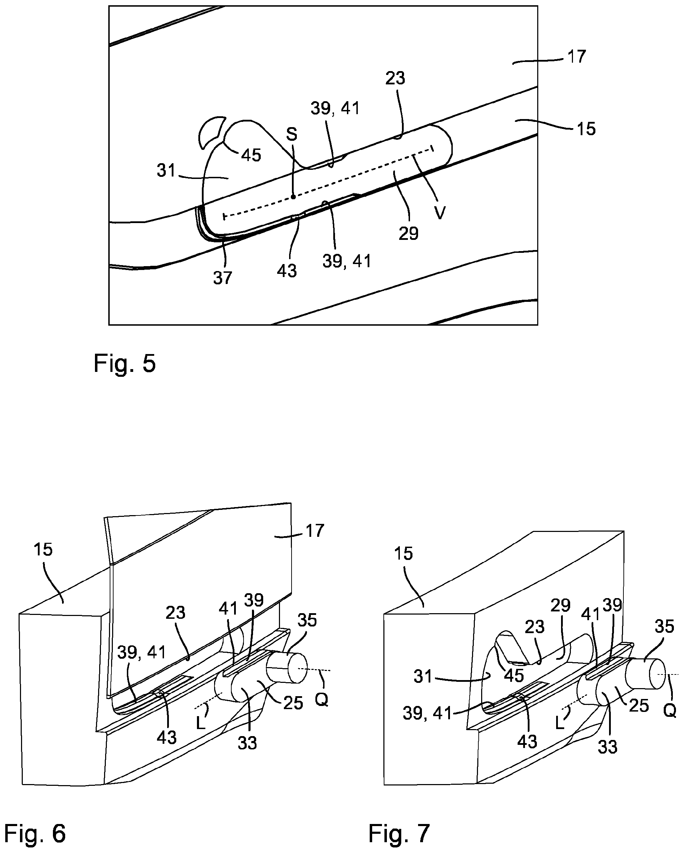

In den Figuren ist eine Ausführungsform eines erfindungsgemäßen Schutzhelmes 11 jeweils in vereinfachter Darstellung gezeigt. Dabei ist der Schutzhelm 11 in den Fig. 1 bis 4 jeweils vollständig aus einer Blickrichtung von der Seite dargestellt. Die Fig. 5 bis 9 zeigen dagegen jeweils lediglich einen Ausschnitt des Schutzhelmes 11 aus mehreren verschiedenen Blickrichtungen.

Bei dem gezeigten Schutzhelm 11 handelt es sich um einen Sporthelm, nämlich um einen Fahrradhelm. Der Schutzhelm 11 umfasst eine stoßabsorbierende Helmschale 13, die zumindest im Wesentlichen eine Halbkugelschalenform aufweist und einen Helmkörper 15 sowie eine dünne Außenschale 17 umfasst. Der Helmkörper 15 kann als Material beispielsweise geschäumtes Polystyrol umfassen, und die Außenschale 17 kann als Material beispielsweise ein AcrylnitrilButadien-Styrol-Copolymer umfassen. Der Schutzhelm 11 umfasst ferner ein Visier 19, das eine länglich gebogene Form mit zumindest im Wesentlichen spitz zulaufenden Enden aufweist. Das Visier 19 ist zumindest teilweise aus einem transparenten Material gebildet.

An einer in den Fig. 1 bis 4 dem Betrachter zugewandten Seite des Schutzhelmes 11 ist an der Helmschale 13 ein Kopplungsabschnitt 21 ausgebildet. Ein weiterer gleichartiger Kopplungsabschnitt 21 ist auch auf der entgegengesetzten Seite des Schutzhelmes 11, die von dem Betrachter weg weist und daher in den Figuren nicht sichtbar ist, an der Helmschale 13 ausgebildet. Zudem weist auch das Visier 19 an seinen beiden entgegengesetzten Enden jeweils einen Kopplungsabschnitt 21 auf, wobei aufgrund der Blickrichtung in den Figuren nur der an dem dem Betrachter zugewandten Ende ausgebildete Kopplungsabschnitt 21 gezeigt ist. Der Kopplungsabschnitt 21 ist dabei mit unterbrochener Linie dargestellt, da er an der vom Betrachter abgewandten Innenseite des Visiers 19 ausgebildet ist.

Die an der Helmschale 13 ausgebildeten Kopplungsabschnitte 21 sind jeweils als Kopplungsaufnahme 23 in Form einer in dem Helmkörper 15 ausgebildeten Ausnehmung ausgebildet. Die an dem Visier 19 ausgebildeten Kopplungsabschnitte 21 sind dagegen als von dem übrigen Visier 19 vorstehende Kopplungsfortsätze 25 ausgebildet.

In Fig. 1 ist das Visier 19 in einem von der Helmschale 13 gelösten Zustand gezeigt. Das Visier 19 kann jedoch je nach Bedarf mit der Helmschale 13 gekoppelt werden, indem auf beiden Seiten des Schutzhelmes 11 jeweils der an dem Visier 19 ausgebildete Kopplungsfortsatz 25 in die an der Helmschale 13 ausgebildete Kopplungsaufnahme 23 eingesetzt wird. Der Kopplungsfortsatz 25 greift dadurch jeweils in einer Eingriffsrichtung, die zumindest im Wesentlichen senkrecht zu einer Außenseite der Helmschale 13 im Bereich der jeweiligen Kopplungsaufnahme 23 bzw. zumindest im Wesentlichen auf einen Mittelpunkt der Halbkugelschalenform der Helmschale 13 zu weisend ausgerichtet ist, in die Kopplungsaufnahme 23 ein.

Grundsätzlich könnten auch gerade umgekehrt die an der Helmschale 13 ausgebildeten Kopplungsabschnitte 21 jeweils als Kopplungsaufnahme 23 ausgebildet sein und die an dem Visier 19 ausgebildeten Kopplungsabschnitte 21 jeweils als Kopplungsfortsatz 25 ausgebildet sein, wobei die Eingriffsrichtung dann gerade entgegengesetzt ausgerichtet wäre.

Für das Anbringen des Visiers 19 kann es erforderlich sein, das Visier 19 gegen eine Eigenelastizität des Visiers 19 aufzuweiten, d. h. seine Enden gegen die genannte Eigenelastizität des Visiers 19 in Richtung voneinander weg auszulenken. Die dabei aus der Eigenelastizität resultierende Rückstellkraft spannt dann die Kopplungsfortsätze 25 in die jeweilige Kopplungsaufnahme 23 vor, so dass das Eingreifen der Kopplungsfortsätze 25 in die Kopplungsaufnahmen 23 unterstützt wird und die Kopplungsfortsätze 25 anschließend durch die Vorspannung in der jeweiligen Kopplungsaufnahme 23 gehalten werden. Die Kopplungsfortsätze 25 können außerdem auch magnetisch in den Kopplungsaufnahmen 23 gesichert sein, wie weiter unten noch erläutert wird.

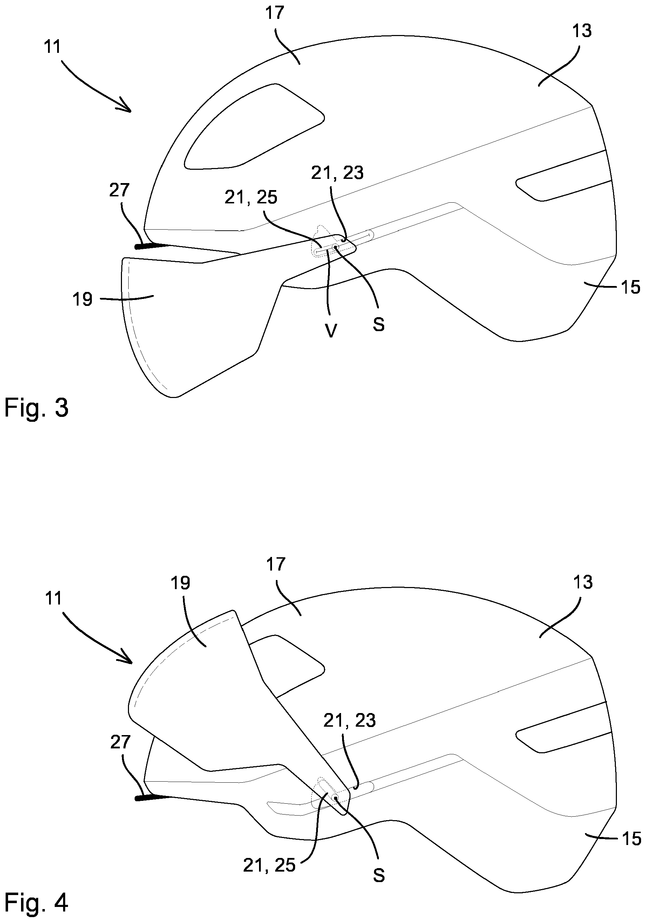

Innerhalb der jeweiligen Kopplungsaufnahme 23 kann der jeweilige Kopplungsfortsatz 25 verschiedene Positionen einnehmen, nämlich insbesondere die in Fig. 2 gezeigte Ausgangsposition, die in Fig. 3 gezeigte Vorschubposition und die in Fig. 4 gezeigte Aufklappposition. Dabei sind die Kopplungsaufnahme 23 und der Kopplungsfortsatz 25 derart relativ zueinander ausgebildet, dass der Kopplungsfortsatz 25 innerhalb der Kopplungsaufnahme 23 aus der Ausgangsposition entlang einer zumindest im Wesentlichen geradlinigen Verschiebestrecke V (vgl. Fig. 2 und 3) in die Vorschubposition verschiebbar und um eine zur Verschiebestrecke V senkrechte Schwenkachse S (vgl. Fig. 3 und 4) aus der Vorschubposition in die Aufklappposition schwenkbar ist sowie umgekehrt aus der Aufklappposition um die Schwenkachse S in die Vorschubposition schwenkbar und aus der Vorschubposition entlang der Verschiebestrecke V in die Ausgangsposition verschiebbar ist.

Diese Beweglichkeit des Kopplungsfortsatzes 25 in der Kopplungsaufnahme 23 aus der Ausgangsposition über die Vorschubposition in die Aufklappposition und zurück aus der Aufklappposition über die Vorschubposition in die Ausgangsposition ist zumindest im Wesentlichen zwangsgeführt, also auf einen einzigen Freiheitsgrad beschränkt. Mit anderen Worten kann der Kopplungsfortsatz 25 zwischen der Ausgangsposition und der Vorschubposition zumindest im Wesentlichen ausschließlich entlang der Schiebestrecke V verschoben werden und zwischen der Vorschubposition und der Aufklappposition zumindest im Wesentlichen ausschließlich um die Schwenkachse S geschwenkt werden. Ferner sind die Kopplungsaufnahme 23 und der Kopplungsfortsatz 25 derart relativ zu einander ausgebildet, dass der Kopplungsfortsatz 25 beim Einsetzen in die Kopplungsaufnahme 23 von den genannten Positionen zwangsläufig als erstes die Ausgangsposition einnimmt und auch umgekehrt zumindest im Wesentlichen ausschließlich in der Ausgangsposition aus der Kopplungsaufnahme 23 entnommen werden kann.

Da die Kopplungsfortsätze 25 mit dem übrigen Visier 19 starr verbunden sind, nimmt das Visier 19 relativ zur Helmschale 13 jeweils eine Stellung ein, die der jeweiligen Position der Kopplungsfortsätze 25 innerhalb der jeweiligen Kopplungsaufnahme 23 entspricht. Wenn die Kopplungsfortsätze 25 die Ausgangsposition einnehmen, befindet sich das Visier 19 in der in Fig. 2 gezeigten Geschlossenstellung; wenn die Kopplungsfortsätze 25 in die Vorschubposition verschoben sind, ist das Visier 19 in entsprechender Weise in die in Fig. 3 gezeigte Stellung vorgeschoben; und wenn sich die Kopplungsfortsätze 25 in der Aufklappposition befinden, nimmt das Visier 19 die in Fig. 4 gezeigte Offenstellung ein.

Der Schutzhelm 11 weist bei der gezeigten Ausführungsform einen an der Helmschale 13 angeordneten Schirm 27 auf, der an einer Vorderseite des Schutzhelmes 11 von der Helmschale 13 vorsteht. In seiner Geschlossenstellung grenzt das Visier 19 in Umlaufrichtung um die Schwenkachse S an den Schirm 27 an. Ist das Visier 19 dagegen vorgeschoben, ist das Visier 19 weiter von der Schwenkachse S entfernt als der Schirm 27, so dass das Visier 19 an dem Schirm 27 vorbei aus der in Fig. 3 gezeigten Stellung in die in Fig. 4 gezeigte Offenstellung geschwenkt werden kann. Diese Art des Verstellens des Visiers 19 ermöglicht es daher, an dem Schutzhelm 11 sowohl einen Schirm 27 als auch ein Visier 19 vorzusehen und den Schutzhelm 11 gleichwohl kompakt auszubilden.

Die Verstellbarkeit des Visiers 19 beruht dabei auf der beschriebenen Beweglichkeit der Kopplungsfortsätze 25 in den Kopplungsaufnahmen 23, die sich insbesondere aus der jeweiligen Form dieser Kopplungsabschnitte 21 ergibt. Die konkrete Ausbildung der Kopplungsaufnahmen 23 und der Kopplungsfortsätze 25 lässt sich insbesondere anhand der Fig. 5 bis 9 nachvollziehen, in denen der in Fig. 1 gekennzeichnete, eine der Kopplungsaufnahmen 23 umfassende Bereich sowie (mit Ausnahme der Fig. 5) auch der mit dieser Kopplungsaufnahme 23 zusammenwirkende Kopplungsfortsatz 25 in Detailansichten gezeigt sind.

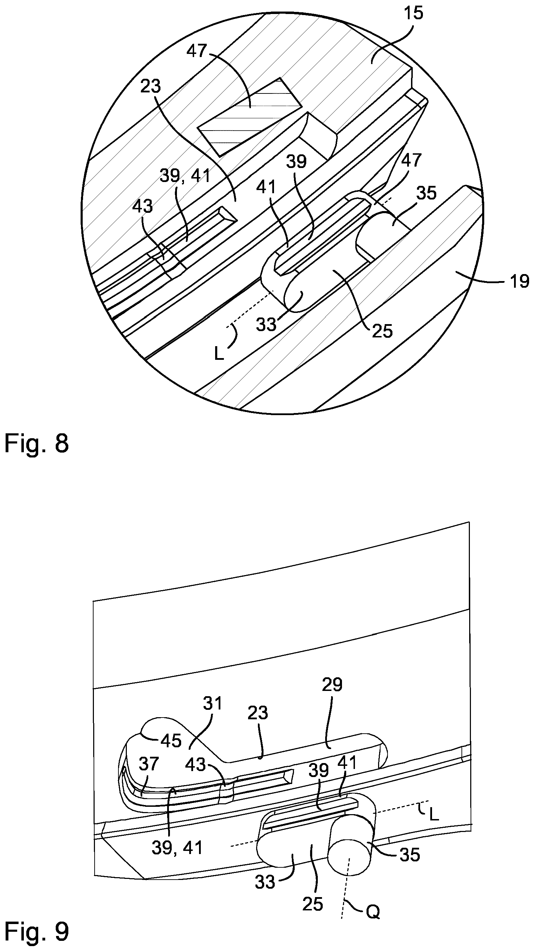

Die Kopplungsaufnahme 23 umfasst einen Verschiebeabschnitt 29, der zumindest im Wesentlichen die Form eines Langlochs aufweist, sowie einen Schwenkabschnitt 31, in den der Verschiebeabschnitt 29 mündet und der zumindest im Wesentlichen die Form eines Kreissektors aufweist (vgl. insbesondere Fig. 5). Der Kopplungsfortsatz 25 umfasst einen Sockelabschnitt 33, der eine längliche Form aufweist und sich entlang einer Längsachse L erstreckt, sowie einen Verbindungsabschnitt 35, der eine Zylinderform aufweist und sich entlang einer zur Längsachse L quer ausgerichteten Querachse Q erstreckt (vgl. insbesondere Fig. 9). Der Verbindungsabschnitt 35 ist dabei an einem Ende der Erstreckung des Sockelabschnitts 33 entlang der Längsachse L mit dem Sockelabschnitt 33 verbunden.

In der Ausgangsposition, in der Vorschubposition und beim Verschieben entlang der Verschiebestrecke ist der Kopplungsfortsatz 25 mit der Längsachse L seine Sockelabschnitts 33 zumindest im Wesentlichen parallel zur Verschiebestrecke V ausgerichtet. Die Zylinderachse der Zylinderform des Verbindungsabschnitts 35 fällt in der Vorschubposition und der Aufklappposition sowie in den dazwischenliegenden Positionen des Kopplungsfortsatzes 25 mit der Schwenkachse S zusammen, um die der Kopplungsfortsatz 25 zwischen der Vorschubposition und der Aufklappposition schwenkbar ist.

Quer zur Verschiebestrecke V wird der Verschiebeabschnitt 29 der Kopplungsaufnahme 23 durch zwei zueinander entgegengesetzte parallele Ränder begrenzt, von denen einer in gerader Verlängerung in einen Rand des Schwenkabschnitts 31 übergeht, der den Schwenkabschnitt 31 in Umlaufrichtung um die Schwenkachse S begrenzt und daher zumindest im Wesentlichen radial zur Schwenkachse S ausgerichtet ist. Dieser Rand des Schwenkabschnitts 31 ist mit zunehmendem Abstand von der Schwenkachse S geringfügig in Richtung des entgegengesetzten Randes des Schwenkabschnitts 31 gebogen und bildet insofern eine Anlaufschräge 37 (vgl. Fig. 5). Infolgedessen wird der Kopplungsfortsatz 25 beim Verschieben entlang der Verschiebestrecke V kurz vor Erreichen der Vorschubposition bereits geringfügig in Richtung der Aufklappposition geschwenkt. Die Verschiebestrecke V ist also nicht exakt geradlinig, sondern weist aufgrund der genannten Anlaufschräge 37 einen Vorschwenkabschnitt auf, der den Übergang zwischen dem Verschieben entlang der Verschiebestrecke V und dem Schwenken um die Schwenkachse S unterstützt.

An den genannten Rändern der Kopplungsaufnahme 23 ist jeweils eine Führungsstruktur 39 vorgesehen, die als Führungssteg 41 ausgebildet ist, der von dem jeweiligen Rand in die Kopplungsaufnahme 23 hinein vorsteht und parallel zur Verschiebestrecke V verläuft. An zueinander entgegengesetzten Rändern des Sockelabschnitts 33, die den Sockelabschnitt 33 quer zu seiner Längsachse L begrenzen und an den genannten Rändern der Kopplungsaufnahme 23 beim Verschieben entlang der Verschiebestrecke V entlanggleiten, ist ebenfalls jeweils eine Führungsstruktur 39 vorgesehen, die als Führungsnut ausgebildet ist.

Beim Verschieben des Kopplungsfortsatzes 25 entlang der Verschiebestrecke V greifen die Führungsstege 41 der an der Kopplungsaufnahme 23 ausgebildeten Führungsstruktur 39 nach Art einer Nut-Feder-Verbindung in die jeweilige an dem entsprechenden Rand des Sockelabschnitts 33 des Kopplungsfortsatzes 25 ausgebildete Führungsnut ein und gleiten darin entlang. Dadurch wird der Kopplungsfortsatz 25 beim Verschieben zwischen der Ausgangsposition und der Vorschubposition entlang der Verschiebestrecke V geführt.

Der von dem Verbindungsabschnitt 35 entferntere Rand der jeweiligen Führungsnut kann dabei auch als Führungssteg 41 betrachtet werden, der beim Verschieben des Kopplungsfortsatzes 25 entlang der Verschiebestrecke V den entsprechenden an der Kopplungsaufnahme 23 ausgebildeten Führungssteg 41 bezüglich der Eingriffsrichtung hintergreift, so dass der Kopplungsfortsatz 25 beim Verschieben entlang der Verschiebestrecke V sowie vorzugsweise auch in der Vorschubposition aufgrund dieses Hintergreifens formschlüssig gegen ein Verlassen der Kopplungsaufnahme 23 entgegen der Eingriffsrichtung gesichert ist.

Auch beim Schwenken des Kopplungsfortsatzes 25 um die Schwenkachse S, bei dem die Führungsstege 41 sich nicht länger gegenseitig hintergreifen, ist der Kopplungsfortsatz 25 formschlüssig gegen ein Verlassen der Kopplungsaufnahme 23 entgegen der Eingriffsrichtung gesichert. Denn der Sockelabschnitt 33 des Kopplungsfortsatzes 25 hintergreift dabei die Außenschale 17 der Helmschale 13 des Schutzhelmes 11 (vgl. Fig. 6).

An einem Rand der Kopplungsaufnahme 23, nämlich an dem Rand, der durch die in gerader Verlängerung ineinander übergehenden Ränder des Verschiebeabschnitts 29 und des Schwenkabschnitts 31 der Kopplungsaufnahme 23 gebildet wird, ist in einem Abschnitt, der der Schwenkachse S am nächsten liegt, eine Stützmulde 43 ausgebildet (vgl. insbesondere Fig. 8). Die Stützmulde 43 ist als eine zur Schwenkachse S parallele längliche Vertiefung in dem Rand ausgebildet. Da an diesem Rand auch einer der genannten Führungsstege 41 ausgebildet ist, erstreckt sich die Stützmulde 43 auch durch diesen Führungssteg 41. Mit anderen Worten ist ein Teil der Stützmulde 43 in diesem Führungssteg 41 ausgebildet. Die Stützmulde 43 ist dabei derart angeordnet, dass sich dasjenige Ende der Längserstreckung des Sockelabschnitts 33 des Kopplungsfortsatzes 25, durch das die Schwenkachse S verläuft (da der Verbindungsabschnitt 35 den Sockelabschnitt 33 an diesem Ende mit dem übrigen Visier 19 verbindet), beim Schwenken um die Schwenkachse S an der Stützmulde 43 abstützt.

An einem weiteren Rand der Kopplungsaufnahme 23, der den Schwenkabschnitt 31 der Kopplungsaufnahme 23 in zur Schwenkachse S radialer Richtung begrenzt, ist ein rückfedernder Rastvorsprung 45 ausgebildet. Der Rastvorsprung 45 ist dabei derart angeordnet, dass er von dem Kopplungsfortsatz 25, nämlich von dem von der Schwenkachse S radial weg weisenden Ende der Längserstreckung des Sockelabschnitts 33 des Kopplungsfortsatzes 25, kurz vor Erreichen der Aufklappposition passiert und dabei vorübergehend zurückgedrängt, d. h. elastisch ausgelenkt, wird. Nach dem Passieren stellt sich der Rastvorsprung aufgrund seiner Elastizität zurück. Dadurch rastet der Kopplungsfortsatz 25 in die Aufklappposition ein und wird danach aufgrund dieses Verrastens in der Aufklappposition gehalten. Allerdings kann der Kopplungsfortsatz 25 durch Aufbringen einer für ein erneutes Zurückdrängen des Rastvorsprungs 45 hinreichend hohen entgegengesetzten Kraft auch wieder in umgekehrter Richtung aus der Aufklappposition an dem Rastvorsprung 45 vorbei zurück in die Vorschubposition geschwenkt werden.

Durch das beschriebene Verrasten des Kopplungsfortsatzes 25 in der Aufklappposition wird insgesamt das Visier 19 gegen ein unbeabsichtigtes Verstellen aus der Offenstellung gesichert.

Bei dem gezeigten Beispiel wird der Rastvorsprung 45 durch einen Abschnitt des genannten Randes des Schwenkabschnitts 31 gebildet, der einen verringerten Radius relativ zur Schwenkachse S aufweist und hinter dem ein Freiraum vorgesehen ist, so dass der Abschnitt dünnwanding ist und elastisch in den Freiraum zurückgedrängt werden kann. Grundsätzlich kann der rückfedernde Rastvorsprung 45 aber auch auf viele verschiedene andere Weisen ausgebildet sein, beispielsweise als eine von dem Rand vorstehende Rastzunge oder Rastfeder. Dabei kann der Rastvorsprung 45 auch von dem Rand separat ausgebildet und an geeigneter Position innerhalb der Kopplungsaufnahme 23 befestigt sein. Zudem braucht der Rastvorsprung 45 nicht unbedingt an dem genannten Rand vorgesehen zu sein, sondern kann beispielsweise auch an einem Boden der Kopplungsaufnahme 23 angeordnet sein.

Der Sockelabschnitt 33 des Kopplungsfortsatzes 25 umfasst ein ferromagnetisches Material und weist insofern ein Magnetelement 47 auf. An dem von dem Schwenkabschnitt 31 beabstandeten Ende der Längserstreckung des Verschiebeabschnitts 29 der Kopplungsaufnahme 23 weist die Kopplungsaufnahme 23 in Form eines in den Helmkörper 15 eingebetteten Permanentmagneten ebenfalls ein Magnetelement 47 auf (vgl. Fig. 8, in der ein Ausschnitt des Schutzhelmes 11 entlang der in Fig. 1 markierten Ebene geschnitten dargestellt ist). Zwischen diesen Magnetelementen 47 entsteht in Abhängigkeit von ihrem Abstand voneinander eine magnetische Anziehung. Bei hinreichend geringem Abstand ergibt sich daraus ein magnetischer Kraftschluss, durch den der Kopplungsfortsatz 25 in die Ausgangsposition gezogen bzw. in der Ausgangsposition gehalten wird.

Auf diese Weise sind die Kopplungsfortsätze 25 nicht nur aufgrund der Eigenelastizität des Visiers 19, sondern noch zusätzlich durch den magnetischen Kraftschluss in der jeweiligen Kopplungsaufnahme 23 gesichert, wenn sie sich in ihrer Ausgangsposition befinden, in der sie grundsätzlich aus der jeweiligen Kopplungsaufnahme 23 entnommen werden können. Dennoch ist ein beabsichtigtes Lösen des Visiers 19 von der Helmschale 13 auf einfache Weise möglich, indem der jeweilige Kopplungsfortsatz 25 mit hinreichender Kraft zur Überwindung des magnetischen Kraftschlusses und entgegen der aus der Eigenelastizität des Visiers 19 resultierenden Vorspannung aus der Kopplungsaufnahme 23 gezogen wird.

Durch das magnetische Zusammenwirken der genannten Magnetelemente 47 wird der jeweilige Kopplungsfortsatz 25 nicht nur gegen ein Lösen aus der Kopplungsaufnahme 23 gesichert, sondern vorteilhafterweise auch dagegen, unbeabsichtigt in die Vorschubposition verschoben zu werden oder zu rutschen. Die Magnetelemente 47 sichern insofern auch das Visier 19 in dessen Geschlossenstellung. Durch Vorsehen eines weiteren Magnetelements 47 im Bereich des genannten Rastvorsprungs 45 (zusätzlich zu diesem oder an dessen Stelle) kann auch das beschriebene Verrasten des Kopplungsfortsatzes 25 in der Aufklappposition durch magnetischen Kraftschluss erfolgen, so dass das Visier 19 auch in seiner Offenstellung magnetisch gesichert ist.

Die beschriebene Art der Kopplung des Visiers 19 mit der Helmschale 13 erlaubt es, das Visier 19 auf einfache Weise je nach Bedarf an der Helmschale 13 anzubringen oder es wegzulassen und es zudem, wenn es an der Helmschale 13 angebracht ist, ohne größeren Aufwand zwischen einer Geschlossenstellung und einer Offenstellung zu verstellen, wobei es zuverlässig an der Helmschale 13 befestigt ist. Die für die Kopplung des Visiers 19 mit der Helmschale 13 vorgesehenen Strukturen, nämlich die Kopplungsfortsätze 25 und die Kopplungsaufnahmen 23, beanspruchen dabei vergleichsweise wenig Platz, so dass der Schutzhelm 11 trotz dieser vorteilhaften Funktionen vergleichsweise schlank ausgebildet sein kann.

### Bezugszeichen

- 11: Schutzhelm
- 13: Helmschale
- 15: Helmkörper
- 17: Außenschale
- 19: Visier
- 21: Kopplungsabschnitt
- 23: Kopplungsaufnahme
- 25: Kopplungsfortsatz
- 27: Schirm
- 29: Verschiebeabschnitt
- 31: Schwenkabschnitt
- 33: Sockelabschnitt
- 35: Verbindungsabschnitt
- 37: Anlaufschräge
- 39: Führungsstruktur
- 41: Führungssteg
- 43: Stützmulde
- 45: Rastvorsprung
- 47: Magnetelement
- L: Längsachse
- Q: Querachse
- S: Schwenkachse
- V: Verschiebestrecke

## Patentansprüche

1. Schutzhelm (11), insbesondere Sporthelm, mit einer stoßabsorbierenden Helmschale (13) sowie einem Visier (19),
wobei an zwei entgegengesetzten Seiten des Schutzhelmes (11) jeweils ein Kopplungsabschnitt (21) der Helmschale (13) und ein Kopplungsabschnitt (21) des Visiers (19) ausgebildet sind, von denen einer als Kopplungsaufnahme (23) und der andere als Kopplungsfortsatz (25) ausgebildet ist und zwar derart, dass der Kopplungsfortsatz (25) in die Kopplungsaufnahme (23) eingreifen kann, um das Visier (19) mit der Helmschale (23) zu koppeln,
wobei der Kopplungsfortsatz (25) in der Kopplungsaufnahme (23) zwischen einer Ausgangsposition und einer Vorschubposition entlang einer Verschiebestrecke (V) verschiebbar und zwischen der Vorschubposition und einer Aufklappposition um eine Schwenkachse (S) schwenkbar ist.

2. Schutzhelm nach Anspruch 1,
wobei die Verschiebestrecke (V) zumindest im Wesentlichen geradlinig ist.

3. Schutzhelm nach Anspruch 1 oder 2,
wobei die Schwenkachse (S) zumindest im Wesentlichen senkrecht zur Verschiebestrecke (V) ausgerichtet ist.

4. Schutzhelm nach einem der vorstehenden Ansprüche,
wobei die Kopplungsaufnahme (23) einen Verschiebeabschnitt (29) umfasst, der zumindest im Wesentlichen die Form eines Langlochs aufweist, und einen Schwenkabschnitt (31) umfasst, der zumindest im Wesentlichen die Form eines Kreissektors aufweist.

5. Schutzhelm nach einem der vorstehenden Ansprüche,
wobei der Kopplungsfortsatz (25) einen Sockelabschnitt (33) mit einer länglichen Form umfasst, der sich entlang einer Längsachse (L) erstreckt und der in der Ausgangsposition, in der Vorschubposition und in der Aufklappposition vollständig in der Kopplungsaufnahme (23) aufgenommen ist, sowie einen Verbindungsabschnitt (35) umfasst, der sich entlang einer zur Längsachse (L) quer ausgerichteten Querachse (Q) erstreckt und den Sockelabschnitt (33) mit dem übrigen Visier (19) bzw. mit der übrigen Helmschale (13) verbindet.

6. Schutzhelm nach Anspruch 5,
wobei der Verbindungsabschnitt (35) an einem Ende der Erstreckung des Sockelabschnitts (33) entlang der Längsachse (L) mit dem Sockelabschnitt (33) verbunden ist.

7. Schutzhelm nach einem der vorstehenden Ansprüche,
wobei der Kopplungsfortsatz (25) beim Verschieben zwischen der Ausgangsposition und der Vorschubposition und/oder beim Schwenken zwischen der Vorschubposition und der Aufklappposition zwangsgeführt ist.

8. Schutzhelm nach einem der vorstehenden Ansprüche,
wobei an der Kopplungsaufnahme (23) eine Führungsstruktur (39) ausgebildet ist, die mit einer an dem Kopplungsfortsatz (25) ausgebildeten Führungsstruktur (39) derart zusammenwirkt, dass der Kopplungsfortsatz (25) beim Verschieben zwischen der Ausgangsposition und der Vorschubposition entlang der Verschiebestrecke (V) geführt wird.

9. Schutzhelm nach Anspruch 8,
wobei die an der Kopplungsaufnahme (23) ausgebildete Führungsstruktur (39) und die an dem Kopplungsfortsatz (25) ausgebildete Führungsstruktur (39) jeweils einen Führungssteg (41) aufweisen und der Führungssteg (41) des Kopplungsfortsatzes (25) den Führungssteg (41) der Kopplungsaufnahme (23) derart hintergreift, dass der Kopplungsfortsatz (25) entlang der Verschiebestrecke (V) formschlüssig gegen ein Verlassen der Kopplungsaufnahme (23) gesichert ist.

10. Schutzhelm nach einem der vorstehenden Ansprüche,
wobei der Kopplungsfortsatz (25) in der Vorschubposition, in der Aufklappposition, entlang der Verschiebestrecke (V) sowie zwischen der Vorschubposition und der Aufklappposition formschlüssig gegen ein Verlassen der Kopplungsaufnahme (23) gesichert ist.

11. Schutzhelm nach einem der vorstehenden Ansprüche,
wobei die Kopplungsaufnahme (23) und der Kopplungsfortsatz (25) jeweils ein Magnetelement (47) aufweisen und die Magnetelemente (47) derart angeordnet sind, dass der Kopplungsfortsatz (25) durch magnetischen Kraftschluss in der Ausgangsposition gehalten wird.

12. Schutzhelm nach einem der vorstehenden Ansprüche,
wobei an einem Rand der Kopplungsaufnahme (23) eine Stützmulde (43) ausgebildet ist, an der sich der Kopplungsfortsatz (25) beim Schwenken zwischen der Vorschubposition und der Aufklappposition abstützt.

13. Schutzhelm nach einem der vorstehenden Ansprüche,
wobei die Kopplungsaufnahme (23) und der Kopplungsfortsatz (25) dazu ausgebildet sind, dass der Kopplungsfortsatz (25) in der Aufklappposition durch Verrasten fixiert ist.

14. Schutzhelm nach einem der vorstehenden Ansprüche,
wobei die Kopplungsaufnahme (23) einen rückfedernden Rastvorsprung (45) aufweist, den der Kopplungsfortsatz (25) beim Schwenken zwischen der Vorschubposition und der Aufklappposition passiert und der von dem Kopplungsfortsatz (25) dabei vorübergehend zurückgedrängt wird.

15. Schutzhelm nach einem der vorstehenden Ansprüche,
wobei die Helmschale (13) einen Schirm (27) aufweist, der, wenn sich die Kopplungsfortsätze (25) in ihrer Ausgangsposition befinden, bezüglich der Schwenkachse (S) im Umlaufrichtung an das Visier (19) angrenzt und radial über das Visier (19) übersteht,
wobei das Visier (19), wenn sich die Kopplungsfortsätze (25) in der Vorschubposition, der Aufklappposition oder dazwischen befinden, radial über den Schirm (27) hinaus von der Schwenkachse (S) beabstandet angeordnet ist.
